(19) Europäisches Patentamt
European Patent Office
Office européen des brevets

(11) **EP 4 083 292 A1**

(12) **EUROPEAN PATENT APPLICATION**
published in accordance with Art. 153(4) EPC

(43) Date of publication:
**02.11.2022 Bulletin 2022/44**

(21) Application number: **20907342.8**

(22) Date of filing: **22.12.2020**

(51) International Patent Classification (IPC):
**D04H 1/728** (2012.01)    **A61K 8/02** (2006.01)
**A61K 8/72** (2006.01)    **A61Q 1/00** (2006.01)

(52) Cooperative Patent Classification (CPC):
**A61K 8/02; A61K 8/72; A61K 8/81; A61Q 1/00;
D04H 1/4382; D04H 1/728**

(86) International application number:
**PCT/JP2020/047982**

(87) International publication number:
**WO 2021/132249 (01.07.2021 Gazette 2021/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.12.2019 JP 2019231573**

(71) Applicant: **Kao Corporation
Chuo-ku
Tokyo 103-8210 (JP)**

(72) Inventors:
• **IKEYAMA, Suguru**
**Haga-gun, Tochigi 321-3497 (JP)**
• **AMARI, Naomi**
**Haga-gun, Tochigi 321-3497 (JP)**
• **SUZUKI, Hiroya**
**Haga-gun, Tochigi 321-3497 (JP)**
• **TOJO, Takehiko**
**Haga-gun, Tochigi 321-3497 (JP)**
• **FUKUDA, Teruyuki**
**Wakayama-shi, Wakayama 640-8404 (JP)**

(74) Representative: **Hoffmann Eitle
Patent- und Rechtsanwälte PartmbB
Arabellastraße 30
81925 München (DE)**

(54) **NANOFIBER SHEET, METHOD FOR USING SAME, AND METHOD FOR MANUFACTURING SAME**

(57)    A nanofiber sheet has a nanofiber layer composed of an accumulation of nanofibers. The nanofiber sheet has a plurality of membrane-like regions which extend between the nanofibers. The membrane-like regions of the nanofiber sheet each contain a water insoluble polymer and a pigment. The water insoluble polymer contained in the membrane-like regions of the nanofiber sheet is different from a constituent member of the nanofibers. The nanofiber sheet is preferred because it has a plurality of membrane-like aggregate regions, each an aggregate of a plurality of membrane-like regions which are adjacent to each other via a fiber and it has a non-membrane-like region between the membrane-like aggregate regions adjacent to each other.

Fig. 3(a)

Example 1

**(Cont. next page)**

Fig. 3(b)

Example 2

Fig. 3(c)

Example 3

Fig. 3(d)

Comparative Example 1

**Description**

Technical Field

The present invention relates to a nanofiber sheet, a method for using same, and a method for manufacturing same.

Background Art

There is known a technique of attaching a nanofiber sheet to the human skin to conceal an uneven color due to redness, freckles, or the like or to improve slight skin roughness such as pores or fine lines. For example, Patent Literature 1 describes a cosmetic sheet for makeup having a nanofiber sheet composed of a polymer compound containing a coloring pigment. This cosmetic sheet for makeup is manufactured by mixing a slurry having a coloring pigment dispersed in a liquid medium and a solution of a polymer compound to prepare a spray liquid and stacking nanofibers by electrospinning with the resulting spray liquid.

Citation List

Patent Literature

Patent Literature 1: US2013/142852A1

Summary of Invention

The present invention relates to a nanofiber sheet having a nanofiber layer having an accumulation of nanofibers.
The nanofiber sheet preferably has a membrane-like region extending between the nanofibers.
The nanofiber sheet preferably has a plurality of the membrane-like regions.
The membrane-like region preferably contains a water insoluble polymer.
The membrane-like region preferably contains a pigment.
The water insoluble polymer is preferably different from a constituent member of the nanofibers.
The present invention also relates to a method of using the aforesaid nanofiber sheet.
The method of using the nanofiber sheet preferably has a step of attaching the nanofiber sheet to an object.
The method of using the nanofiber sheet preferably has a step of applying a liquid to the nanofiber sheet or the object.
The present invention also relates to a method of manufacturing a nanofiber sheet including forming a nanofiber layer having an accumulation of nanofibers by electrospinning.
A colored region is preferably formed in the nanofiber layer by performing printing on the nanofiber layer by inkjet printing with an ink containing a water insoluble polymer and a pigment.
The water insoluble polymer is preferably different from a constituent member of the nanofibers.
The colored region has preferably a color different from a ground color of the accumulation of the nanofibers.
Other characteristics of the present invention will be apparent from claims and the following description.

Brief Description of Drawings

[Fig. 1] Figs. 1(a) to (c) are schematic views showing the modes of membrane-like regions, respectively, and Fig. 1(d) is a schematic view showing the mode of a membrane-like aggregate region.
[Fig. 2] Fig. 2(a) is a photographic image of the nanofiber sheet obtained in Example 1, Fig. 2(b) is a photographic image of the nanofiber sheet obtained in Example 2, Fig. 2(c) is a photographic image of the nanofiber sheet obtained in Example 3, and Fig. 2(d) is a photographic image of a nonwoven fabric obtained in Comparative Example 1.
[Fig. 3] Fig. 3(a) is a fragmentary enlarged micrograph of Fig. 2(a), Fig. 3(b) is a fragmentary enlarged micrograph of Fig. 2(b), Fig. 3(c) is a fragmentary enlarged micrograph of Fig. 2(c), and Fig. 3(d) is a fragmentary enlarged micrograph of Fig. 2(d).
[Fig. 4] Fig. 4 is a fragmentary enlarged micrograph of Fig. 3(a).
[Fig. 5] Fig. 5 is a photographic image of the nanofiber sheet obtained in Example 5.

Description of Embodiments

When an upper limit, a lower limit, or both upper and lower limits of a value are specified herein, they include the upper limit or lower limit itself. It is construed that even if not described specifically, all the values not more than the upper limit or not less than the lower limit or all the numerical ranges within the upper and lower limits are described.

In the present specification, "a" and "an" are construed as having a meaning of "one or more".

It can be understood from the above disclosure and the following disclosure in the present specification that various changed or modified modes of the present invention are possible. It should therefore be understood that within the technical scope based on the description of claims, the present invention can be applied to embodiments not specified herein.

All the details described in the aforesaid patent literature and the following patent literature are incorporated herein as a part of the details of the present specification.

The present application claims priority from Japanese Patent Application No. 2019-231573 filed on December 23, 2019 and all the details described in Japanese Patent Application No. 2019-231573 are incorporated herein as a part of the present specification.

The cosmetic sheet for makeup described in Patent Literature 1 has such an advantage that since it is colored in a predetermined color by a coloring pigment, there is a high sense of unity between the cosmetic sheet and the skin to which it is attached. In the principle of electrospinning, however, it is difficult to manufacture a nanofiber sheet colored in a plurality of colors or a nanofiber sheet colored in various shades of colors. In addition, it is also difficult to color a limited region of the sheet.

The present invention therefore relates to an improvement in a colored nanofiber sheet, more specifically relates to a nanofiber showing good color development at a colored site in a nanofiber sheet, and also relates to a method capable of easily manufacturing such a nanofiber sheet.

The present invention will hereinafter be described based on preferred embodiments of it. The present invention relates to a nanofiber sheet. The nanofiber sheet of the present invention has a nanofiber layer.

The nanofiber sheet may be composed solely of a nanofiber layer or, as described later, may be composed of a laminate of a nanofiber layer and a layer other than the nanofiber layer. Examples of the layer other than the nanofiber layer may be one or two or more selected from fiber layers, adhesive layers, and films.

The nanofiber layer is equipped with an accumulation of nanofibers. The term "nanofiber" generally means a fiber having a size, in terms of a circle equivalent diameter, of 10 nm or more and 3000 nm or less. The size of the nanofibers is determined, for example, by enlarging the fibers to 10000 times and observing them through scanning electron microscopic observation, arbitrarily selecting 10 fibers except defective ones (for example, wads of fibers, intersections of fibers, and polymer droplets) from the two-dimensional image, drawing lines orthogonal to the longitudinal direction of the fibers, and directly reading a fiber diameter.

The nanofiber layer is desirably composed solely of nanofibers, but the nanofiber layer is allowed to slightly contain fibers having a size larger than that of nanofibers, wads of nanofibers, coagulated polymer droplets, and the like, each inevitably mixed in during the manufacture of nanofibers.

Due to the bleed out of raw materials constituting the nanofibers (for example, an oily component such as silicone oil), the nanofibers constituting the nanofiber layer may have some of the raw materials on the surface of the nanofibers. Such nanofibers are also embraced in the nanofibers of the present invention.

In the nanofiber layer, nanofibers are stacked at random. As a result, the nanofiber layer is a porous member. The nanofiber layer therefore has air permeability or liquid permeability.

The nanofiber layer practically has a thickness of 50 $\mu$m or less. In particular, it preferably has the aforesaid thickness or less from the standpoint that when the sheet is attached to the skin, the sheet follows the skin and therefore, it does not cause a discomfort even if it is attached to the skin. To make this advantage more outstanding, the nanofiber layer has more preferably a thickness of 35 $\mu$m or less, even more preferably 20 $\mu$m or less.

On the other hand, the nanofiber layer practically has a thickness of 1 $\mu$m or more. In particular, when a base layer and the nanofiber layer are stacked one after another, the nanofiber layer preferably has the aforesaid thickness or less from the standpoint of easy release of the base layer from the nanofiber layer. To make this advantage more outstanding, the nanofiber layer has more preferably a thickness of 3 $\mu$m or more, even more preferably 5 $\mu$m or more.

Comprehensively, the nanofiber layer preferably has a thickness of 1 $\mu$m or more and 50 $\mu$m or less, more preferably 3 $\mu$m or more and 35 $\mu$m or less, even more preferably 5 $\mu$m or more and 20 $\mu$m or less.

The term "thickness of the nanofiber layer" means a thickness of a nanofiber layer which is one of nanofiber layers constituting the nanofiber sheet of the present invention and has a membrane-like region, which will be described later, formed in the layer. The thickness of the nanofiber layer is measured using a contact type film thickness meter [Litematic VL-50A, product of Mitutoyo Corporation (R5 mm carbide spherical surface probe)]. At the time of measurement, a load to be applied to a measuring object is 0.01 Pa.

The nanofiber layer practically has a basis weight of 8 g/m$^2$ or less. In particular, when a liquid is applied to the nanofiber layer, the basis weight is preferably not more than the aforesaid one from the standpoint that the nanofiber layer to which a liquid is applied is likely to be transparent and a colored region, which will be described later, formed in the nanofiber layer visually becomes clearer. To make this advantage more outstanding, the nanofiber layer has more preferably a basis weight of 6 $g/m^2$ or less, even more preferably 4 $g/m^2$ or less.

On the other hand, the nanofiber layer preferably has a basis weight of 0.01 $g/m^2$ or more from the standpoint that such a nanofiber layer can bear a colorant, which will be described later, in a membrane-like region to an easily visible extent. To make this advantage more outstanding, the nanofiber layer has more preferably a basis weight of 0.1 $g/m^2$ or more, even more preferably 1 $g/m^2$ or more.

Comprehensively, the nanofiber layer preferably has a basis weight of 0.01 $g/m^2$ or more and 8 $g/m^2$ or less, more preferably 0.1 $g/m^2$ or more and 6 $g/m^2$ or less, even more preferably 1 $g/m^2$ or more and 4 $g/m^2$ or less.

The term "basis weight of the nanofiber layer" means the basis weight of a nanofiber layer which is one of nanofiber layers constituting the nanofiber sheet of the present invention and has a membrane-like region, which will be described later, formed in the layer. The basis weight of the nanofiber layer is calculated by cutting a 10 mm $\times$ 10 mm measurement piece from the nanofiber layer, weighing the mass of the measurement piece, and multiplying it by 10 000.

A nanofiber is usually composed of a polymer. This polymer may be either water soluble or water insoluble. The term "nanofiber composed of a polymer" does not exclude a nanofiber having a polymer as a main component and also containing another component.

Whether a water soluble or water insoluble nanofiber is used may be determined depending on the specific intended use of the nanofiber sheet of the present invention. For the use of the nanofiber sheet of the present invention, for example, as a cosmetic sheet for makeup to be attached to the human skin, the nanofiber is preferably water insoluble. Examples of the intended use of the nanofiber sheet to be attached to the human skin include cosmetic sheets for makeup, decorative sheets, sheets for special effects makeup, sheets for body art, and skin protective sheets.

In the nanofiber layer, nanofibers are stacked at random as described above and therefore they have a space therebetween. In the present invention, a membrane-like region is present in this space.

The membrane-like region preferably contains a water insoluble polymer. Even if the nanofiber sheet of the present invention is brought into contact with water, such a membrane-like region does not dissolve in water and the membrane-like region can maintain its form. The membrane-like region is allowed to contain a water soluble polymer in addition to the water insoluble polymer unless it dissolves in water and disappears.

The membrane-like region is composed of a polymer different from the constituent member of the nanofiber, for example, a polymer constituting the nanofiber. The term "polymer different from the constituent member of the nanofiber" means a polymer different in at least one of the kind of a side chain, the kind of a terminal group, a molecular weight distribution, an average molecular weight, the presence or absence of a branch, the number of the branch, the presence or absence of a crosslink, a degree of crosslinking, and the kind of a monomer unit of the main chain of the polymer, and when the polymer is composed of a plurality of monomer units, a composition ratio of the monomer units.

Whether the polymer constituting the membrane-like region is different from the polymer constituting the nanofiber is determined by analyzing samples obtained by collecting many sites containing many membrane-like regions and samples obtained by collecting sites not containing a membrane-like region by known techniques such as NMR (a nuclear magnetic resonance apparatus), chromatography, and IR analysis, or by a combination of them and identifying the kind of a side chain, the kind of a terminal group, a molecular weight distribution, an average molecular weight, the presence or absence of a branch, the number of the branch, the presence or absence of a crosslink, a degree of crosslinking, and the kind of a monomer unit of the main chain of the polymer, and when the polymer is composed of a plurality of monomer units, a composition ratio of the monomer units. The measurement of the samples obtained by collecting sites not containing a membrane-like region is performed at 5 points or more at respectively different sites and a range of the measurement value of the polymer component at a site not containing a membrane-like region is calculated. Then, when the measurement value of the polymer component constituting the membrane-like region includes a component outside the range of the measurement value of the polymer component at a site not containing the membrane-like region, the polymer constituting the membrane-like region is found to be different from the polymer constituting the nanofiber.

Whether the polymer having a membrane-like region is a "water insoluble polymer" or not is found as follows. A 7 mm $\times$ 5 mm measurement piece having a colored region, which will be described later, is cut from a nanofiber sheet and the resulting measurement piece is magnified with a microscope or the like and photographed. An area of a colored region (which will hereinafter be called "area A") is measured from the resulting photographic image. After another nanofiber sheet is immersed in 100 mL of deionized water at 25°C for 5 minutes, it is taken out from the deionized water. A measurement piece obtained by cutting it into a size equal to the aforesaid one is left to stand for 24 hours to dry. The measurement sample thus dried is magnified with a microscope or the like and photographed. Based on the resulting photographic image, an area of a colored region is measured (which will hereinafter be called "area B"). When a ratio of a change in area in percent before and after immersion in deionized water [{(area B after immersion) / (area A before immersion)} × 100] is 20% or more, the polymer which the membrane-like region has is determined to be a water insoluble polymer.

On the other hand, the "water soluble polymer" is determined as follows. Measurement is performed in a manner similar to that described above and a ratio of a change in area in percent before and after immersion in deionized water [{(area B after immersion) / (area A before immersion)} × 100] is less than 20%, the polymer which the membrane-like region has is determined to be a water soluble polymer.

Whether the membrane-like region is composed of a polymer or not is determined by elemental analysis using SEM-EDS prior to the judgement by the aforesaid method on whether the membrane-like region is water insoluble or not. When the results of elemental analysis show that the membrane-like region is composed of C, O, and H, the membrane-like region is determined to be composed of a polymer.

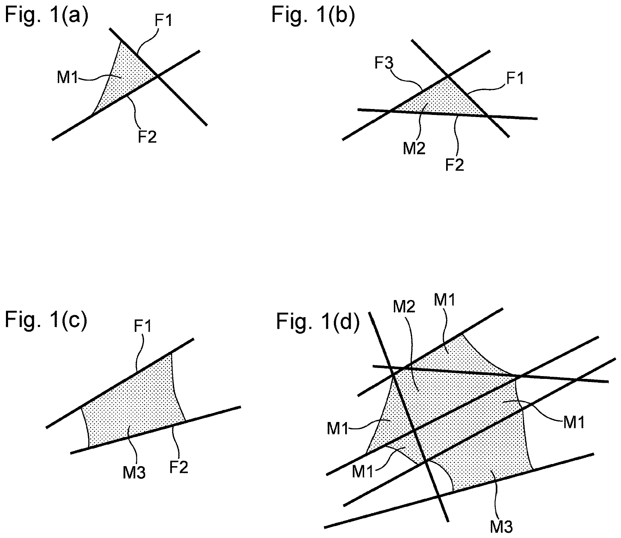

The membrane-like region is formed to extend between the nanofibers as shown in Fig. 1. For example, (A) a membrane-like region M1 extends to include an intersection between two nanofibers F1 and F2 and bridge between the nanofibers as shown in Fig. 1(a), (B) a membrane-like region M2 extends over a closed space defined by three or more nanofibers F1, F2, and F3 as shown in Fig. 1(b), or (C) a membrane-like region M3 extends to bridge between two nanofibers F1 and F2 as shown in Fig. 1(c).

When the nanofiber layer is viewed along the thickness direction thereof, the membrane-like region may be present at any position in the thickness direction. For example, the membrane-like region may be present only at one of the surfaces of the nanofiber layer, may be present only at both surfaces of the nanofiber layer, or may be present only at a center site in the thickness direction of the nanofiber layer. The membrane-like region may be present in the entire region in the thickness direction from one of the surfaces of the nanofiber layer to the other surface. Of these modes, the membrane-like region, which presents in the entire region in the thickness direction from one of the surfaces of the nanofiber layer to the other surface, is preferred from the standpoint that a colored region which will be described later becomes clearer.

The membrane-like region may be present in any of the aforesaid modes (A) to (C) or in another mode. The membrane-like region may also be present as a membrane-like aggregate region which is a combination of two or more modes selected from the aforesaid (A) to (C) modes and the another mode (refer to Fig. 1(d)). The term "membrane-like aggregate region" means a region in which two or more aforesaid membrane-like regions are adjacent to each other via a fiber and constitute an aggregate.

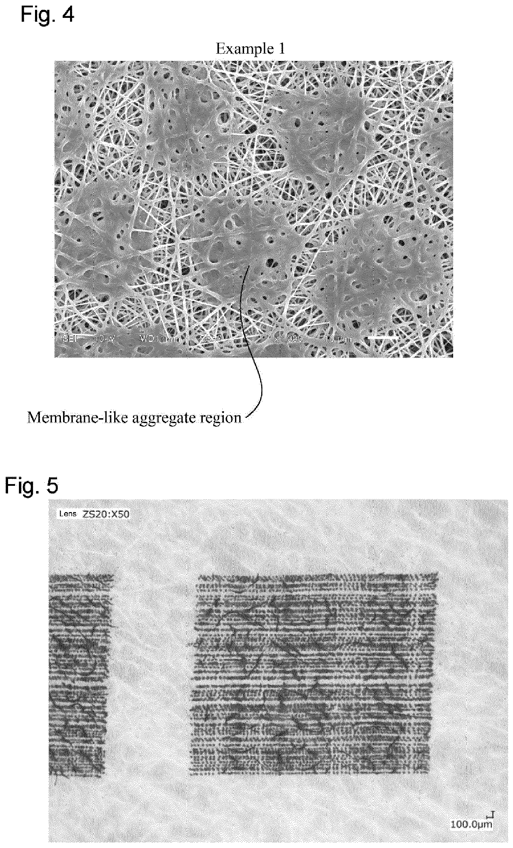

The nanofibers constituting the nanofiber layer are stacked at random, so that the membrane-like region has a random shape. On the other hand, the membrane-like aggregate region may have a random shape or may have a certain shape in a planar view. When a plurality of membrane-like aggregate regions has a certain shape, the membrane-like aggregate region has, for example, a dot-like shape in planar view. For example, in the nanofiber sheet obtained in Example 1 which will be described later, a plurality of membrane-like aggregate regions is formed in the nanofiber layer as shown in Fig. 4. The membrane-like aggregate regions each have a substantially dot-like shape.

The membrane-like aggregate regions are independently present each other. Two or more membrane-like regions are placed without a space and form the aforesaid membrane-like aggregate region. The aforesaid membrane-like aggregate regions are formed to place a space, that is, a non-membrane-like region between membrane-like aggregate regions adjacent to each other. Due to the presence of the non-membrane-like region, the nanofiber layer having a plurality of membrane-like aggregate regions has air permeability. As a result, when the nanofiber sheet of the present invention is attached to, for example, the skin, it has such an advantage that it hardly steams up or does not disturb the cutaneous respiration.

The dimension of the plurality of membrane-like aggregate regions can be set as needed, depending on the specific intended use of the nanofiber sheet of the present invention. From the standpoint of imparting the sheet with a natural color and transparency, the dimension of the membrane-like aggregate region is preferably 2 800 $\mu m^2$ or less in terms of an average area in a planar view from the standpoint of making a colored region, which will be described later, clear. It is more preferably 1 600 $\mu m^2$ or less, even more preferably 1 350 $\mu m^2$ or less.

The lower limit of the average area of the membrane-like aggregate region is preferably 300 $\mu m^2$ when the membrane-like aggregate region is formed by inkjet printing which will be described later.

When the membrane-like aggregate regions are present independently, the aforesaid average area can be calculated, for example, by performing scanning type electron microscopic observation to magnify the membrane-like aggregate region 1 000 times and observe it, arbitrarily selecting five positions from the two-dimensional image, directly reading the circle equivalent diameter of them, calculating the circle equivalent area from the diameter, and determining the average. When the membrane-like aggregate regions adjacent to each other are partially bound, the circle equivalent diameter is read from the unbound portion and then, the average area can be calculated in a similar manner to that described above when the membrane-like aggregate regions are present independently.

The water insoluble polymer contained in the membrane-like region is preferably different from the polymer constituting the nanofiber.

The water insoluble polymer contained in the membrane-like region is preferably a polymer having a function, as a binder, of binding to the nanofiber and a function of dispersing a pigment and from such a standpoint, two or more polymers are preferably used.

Examples of the water insoluble polymer include one or two or more selected from polyester-based resins, polyolefin-based resins, polyamide-based resins, fluorine-based resins, acrylic resins, styrene-based resins, urethane-based resins, vinyl-based resins, imide-based resins, silicone-based resins, and natural resins.

Examples of the polyester-based resins include one or two or more selected from polyethylene terephthalate (PET), polytetramethylene terephthalate, polybutylene terephthalate (PBT), polyethylene naphthalate (PEN), polybutylene naphthalate, polyhydroxybutyric acid (PHB), polyhydroxyalkanoate (PHA), polybutylene succinate (PBS), polylactic acid (PLA), and polybutylene naphthalate (PBN).

Examples of the polyolefin-based resins include one or two or more selected from polypropylene and polyethylene.

Examples of the polyamide-based resins include one or two or more selected from nylon and aramid.

Examples of the fluorine-based resins include one or two or more selected from polytetrafluoroethylene resin (PTFE), polychlorotrifluoroethylene (PCTFE, CTFE), polyvinylidene fluoride (PVDF), polyvinyl fluoride (PVF), perfluoroalkoxyfluoroplastics (PFA), and tetrafluoroethylene hexafluoropropylene copolymer (FEP).

Examples of the vinyl-based resins include one or two or more selected from vinyl chloride resins, vinyl acetate resins, vinylidene chloride resins, completely saponified polyvinyl alcohol which can be insolubilized after fiber formation, partially saponified polyvinyl alcohol which can be subjected to a crosslinking treatment after fiber formation when a crosslinking agent is used in combination, polyvinyl acetal, polyvinyl butyral, and polyvinyl acetal diethylamino acetate.

Examples of the imide-based resins include one or two or more selected from polyimide resins and polyamide-imide resins.

Examples of the silicone-based resins include one or two or more selected from silicone resins, and oxazoline-modified silicone resins such as poly(N-propanoylethyleneimine)-graft-dimethylsiloxane/y-aminopropylmethylsiloxane copolymer.

Examples of the natural resins include one or two or more selected from Twain (main component of corn protein), rosin, shellac, collagen, gelatin, fibrin, and casein.

As the polymer contained in the membrane-like region, particles like those in an emulsion form are usable and the aforesaid polymer are also usable. More preferred examples include one or two or more selected from homopolymers of an acrylic monomer, copolymers of two or more acrylic monomers, and copolymers of an acrylic monomer with another monomer. In this case, examples of the acrylic monomer include acrylic acid, alkyl acrylates such as methyl acrylate, ethyl acrylate, acrylamide, n-propyl acrylate, n-butyl acrylate, isobutyl acrylate, octyl acrylate and 2-ethylhexyl acrylate, acrylic acid 2N,N-dimethylaminoethyl acrylate, acrylonitrile, methacrylic acid, ethyl methacrylate, methacrylamide, n-propyl methacrylate, n-butyl methacrylate, isobutyl methacrylate, 2-ethylhexyl methacrylate, octyl methacrylate, hydroxyethyl methacrylate, and N,N-dimethylaminoethyl methacrylate. Examples of the another monomer copolymerizable with the acrylic monomer include styrene, vinyl acetate, silicone macromers, fluorine-based monomers, and alkoxysilane unsaturated monomers. One or two or more monomers selected from these monomers are usable.

The nanofiber sheet of the present invention has a colored region. The colored region is composed of a plurality of membrane-like aggregate regions. When the membrane-like aggregate regions are each, for example, in a dot shape, the colored region is made of a plurality of dots. The colored region contains any one of a letter, a graphic, and a symbol. The term "letter" means a language or speech sound symbolized into a visible form. The term "graphic" means a part of a plane having a shape combined into one and including a pattern painted in different colors or a pattern colored with different shades. The term "symbol" means a sign other than the letter. As described above, the plurality of membrane-like aggregate regions has a color different from the ground color of the nanofiber layer, so that the colored region in which the plurality of membrane-like aggregate regions has gathered is perceived as a region having a color different from the ground color of the nanofiber layer, by which any one or more of the letter, graphic, and symbol can be recognized by the naked eye.

When the colored region of the nanofiber sheet of the present invention is observed macroscopically, it has a color different from the ground color of the accumulation of nanofibers. The term "observed macroscopically" means that for example, the nanofiber sheet is recognized by the naked eye without magnifying it with a microscope or the like. A ground color of the accumulation of nanofibers is a color derived from the nanofibers themselves constituting the accumulation of nanofibers. This ground color is a color which the nanofiber layer has when no coloring treatment is performed to the accumulation of nanofibers formed. For example, when the nanofiber is made of a synthetic resin and the synthetic resin does not contain a coloring material, the ground color of the accumulation of nanofibers is generally white or milky white (semitransparent white). When the nanofiber is made of a synthetic resin and the synthetic resin contains a coloring material, the ground color of the accumulation of nanofibers is a color derived from the coloring material.

The term "color different from the ground color of the accumulation of nanofibers" means that a difference in RGB value, that is, ΔRGB between the ground color of the accumulation of nanofibers and the color of a colored region which will be described later is 100 or more. The difference in RGB value is determined as follows. The RGB value at a position X1 of the colored region is measured as (R1,G1,B1) and the RGB value at a position X2 of the accumulation of nanofibers (that is, a position of the ground color) is measured as (R2,G2,B2) and the difference is determined from the following formula (1).

$$\Delta RGB = \sqrt{((R1\text{-}R2)^2 + (G1\text{-}G2)^2 + (B1\text{-}B2)^2)} \qquad (1)$$

The RGB used in the calculation of $\Delta RGB$ can be measured with a microscope VHX-5000, product of Keyence Corporation. With this apparatus, the RGB values at X1 and X2 are measured from an image obtained at a magnification of 50. Since the RGB value differs depending on an illumination intensity, a green color is selected from 9 colors, that is, 3 × 3 colors of an image correction color chart CASMATCH (CA010V001-1) and is used as a color bouncer. The measurement is conducted under the conditions of an illumination intensity in which the RGB value of this green color falls within a range of from (110, 200, 120) to (190, 255, 210).

The nanofiber sheet of the present invention can be used as a cosmetic sheet for makeup which is used, for example, for makeup. In this case, the cosmetic sheet for makeup can be utilized in various scenes, depending on the mode of the colored region. For example, when an attention is paid to one colored region, a nanofiber sheet having the colored region with a plurality of colors or a nanofiber sheet having patterns colored with different shades is suited for use as a cosmetic sheet for makeup, for example, as a point makeup of the skin or as a blotch concealer. On the other hand, a nanofiber sheet having a colored region made of a letter or graphic is suited as a cosmetic sheet for makeup, for example, for face painting or body painting.

The color of the colored region comes from the membrane-like region. Described specifically, the membrane-like region contains a coloring material, so that a membrane-like aggregate region which is an aggregate of a plurality of the membrane-like regions has a color derived from the coloring material and therefore, the colored region comprised of an aggregate of a plurality of the membrane-like aggregate regions also has a color derived from the coloring material. In a plurality of membrane-like aggregate regions constituting a colored region, for example, a plurality of membrane-like aggregate regions having a dot shape, the membrane-like aggregate regions adjacent to each other are preferably separated from each other because the colored region can be visually recognized more clearly.

As the coloring material, one or two or more coloring materials selected from pigments and dyes can be used. Pigments are preferred from the standpoint of making the color of the colored region more clear and easy coloring. In this case, since the membrane-like region contains a pigment, the colored region can have a color derived from the pigment.

The term "pigment" means a substance having a property of coloring an object therewith and insoluble in water and an oil. By using the pigment as the coloring material, the colored region can be effectively prevented from blurring even if the nanofiber sheet of the present invention is wetted with a liquid, particularly water.

Examples of the pigment include inorganic and organic pigments and powders. Examples of the organic pigments include natural coloring agents, synthetic monomer coloring agents, and synthetic polymer coloring agents. Examples of the inorganic pigments include talc, kaolin, mica, sericite, phlogopite, magnesium aluminum silicate, calcium silicate, aluminum silicate, magnesium silicate, barium silicate, strontium silicate, epoxy coated aluminum, aluminum powder, calcium phosphate, silicic anhydride, anhydrous aluminum silicate, pyrophyllite clay, bentonite, smectite, montmorillonite, vermiculite, hectorite, zeolite, heidilite, silica, alumina, zirconia, iron oxides (red oxide, yellow iron oxide, black iron oxide, and $\gamma$-iron oxide), yellow ocher, black titanium oxide, sub-stoichiometric titanium oxide, iron titanium oxide, zinc oxide, aluminum hydroxide, aluminum oxide, cobalt aluminum oxide, chromium oxide, zirconium oxide, titanium oxide, titanium oxide sol, sintered iron oxide-titanium dioxide, cerium oxide, magnesium oxide, chromium hydroxide, sintered titanium-titanium dioxide, cobalt titanate, manganese violet, cobalt violet, calcium carbonate, barium carbonate, magnesium carbonate, metal salts of tungstic acid, barium sulfate, calcined calcium sulfate (calcined gypsum), bismuth oxychloride, calamine, sodium rosinate-treated magnesium oxide, fluorapatite, hydroxyapatite, ceramic powder, and metal soaps (such as zinc myristate, calcium palmitate, and aluminum stearate), and a composite powder of two or more pigments selected therefrom. Further, organic-inorganic composite powders such as acrylic resin-coated aluminum powder and titanium oxide-coated nylon powder are also usable.

All the membrane-like regions are not required to contain the same pigment and the pigments contained in the respective membrane-like regions may be different from each other. The concentrations of the pigment contained in the respective membrane-like regions are not required to be the same and the concentrations of the pigment contained in the respective membrane-like regions may be different from each other. Further, the kind of the pigment contained in the membrane-like region is not limited to one and two or more pigments may be contained.

In order to identify the pigment, the kind and amount of elements contained in the pigment may be analyzed by the element mapping of a colored portion by using a known analyzing apparatus such as SEM-EDS or X-ray fluorimetry. In addition, the pigment may be identified by using, singly or in combination, methods such as identification of the kind of a compound based on a detected peak position obtained by analyzing a colored portion by X-ray diffraction (XRD) analysis and analysis of the content of each compound based on the intensity of the detected peak thus measured.

In the nanofiber sheet, a ratio of the coloring material contained in the membrane-like region to the water insoluble polymer contained in the membrane-like region is preferably adjusted to fall within a specific range. By doing so, the colored region composed of an aggregate of a plurality of membrane-like aggregate regions can be made clearer.

From the standpoint of preventing an increase in the viscosity of an ink, a ratio of the mass of the coloring material contained in a membrane-like region to the mass of the water insoluble polymer contained in the membrane-like region (this ratio will hereinafter also be called "a (coloring material)/(polymer) ratio") is preferably 1 or more, more preferably 1.5 or more.

From the standpoint of keeping dispersion stability of the pigment and facilitating the discharge of an ink, the (coloring material)/(polymer) ratio is preferably 5.7 or less, more preferably 3 or less. In particular, the (coloring material)/(polymer) ratio is more preferably 1 or more and 5.7 or less, even more preferably 1.5 or more and 3 or less.

As described above, in the nanofiber sheet of the present invention, nanofibers constituting a nanofiber layer have colored membrane-like regions therebetween, a plurality of the membrane-like regions gather to form a membrane-like aggregate region, and a plurality of the membrane-like aggregate regions gather to form a colored region. The colored region constitutes a letter, a graphic, or a symbol. As is apparent from a preferred method of manufacturing a nanofiber sheet according to the present invention which will be described later, the colored region is not formed by coloring the nanofibers which constitute the nanofiber layer, so that the colored region can be formed with a very large degree of freedom. According to the present invention, therefore, a colored region with a plurality of colors or a colored region with different shades can be formed easily. A colored region can also be formed easily only in a specific region in the nanofiber layer. If there is a colored region derived from the membrane-like region, the nanofibers themselves constituting the nanofiber layer are not prevented from being colored.

When a colored region is formed in the nanofiber layer, the colored region is preferably not placed in a peripheral region of a predetermined width which includes the periphery of the nanofiber layer and extends inward from the periphery. For example, it is preferred that the nanofiber layer includes a center region comprised of the colored region and a non-colored region surrounding the colored region and the non-colored region is a peripheral region of a predetermined width which includes the periphery of the nanofiber layer and extends inward from the periphery. Using a nanofiber sheet equipped with the nanofiber layer having such a mode, for example, as a cosmetic sheet for makeup to be attached to the skin is advantageous because the boundary between the nanofiber layer and the skin is hard to be found visually and the nanofiber layer looks natural as if it is a part of the skin.

To prevent the colored region from being placed in the peripheral region, a manufacturing method using an electrospinning step and a printing step in combination can be used. Described specifically, in the electrospinning step, a nanofiber layer having a peripheral region and a center region is formed using an electrospinning apparatus having a moving mechanism in which a nozzle moves in a surface direction and the position data of the peripheral region and the position data of the center region are recorded based on the data of the moving track of the nozzle. Then, in the printing step, based on the aforesaid position data, printing is performed only on the center region so as not to provide a colored region in the peripheral region.

Various methods can be used for the formation of the membrane-like region and then, the colored region in the nanofiber layer.

As a result of the study by the present inventors, it has been found that the membrane-like region can be formed more successfully by using inkjet printing for the formation of the membrane-like region. Inkjet printing is a method of converting an ink into very minute droplets and spraying the resulting droplets directly to a recording medium.

For the formation of the membrane-like region by inkjet printing, an ink containing a water insoluble polymer is preferably used. The water insoluble polymer is preferably different from the constituent member of the nanofiber. Using this ink facilitates the formation of a membrane-like region containing the water insoluble polymer. It is also preferred that the ink contains two or more water insoluble polymers because of the aforesaid reasons. The water insoluble polymer may be dissolved in the medium of an ink or may be dispersed in the form of fine particles as in an emulsion. The ink may contain a coloring material. Using a pigment as the coloring material facilitates the formation of a membrane-like region containing the pigment.

By conducting inkjet printing on a nanofiber layer with the ink containing the water insoluble polymer and the coloring material, a colored region different from the accumulation of nanofibers can be formed in the nanofiber layer. Typical inkjet printing methods known to date are a piezoelectric method in which ink droplets are formed making use of the deformation of piezo elements and a thermal method in which an ink having bubbles formed therein by heating are jetted. Either printing method can be used in the present invention.

By discharging and spraying an ink to a nanofiber layer by inkjet printing, ink droplets adhere to the component fibers of the nanofiber layer and as shown in Fig. 1(a) to Fig. 1(c), a membrane-like region is formed to bridge between the fibers.

From the standpoint of maintaining the dispersion stability of the pigment and facilitating discharging of the ink, the concentration of the water insoluble polymer contained in the ink is preferably 0.5 mass% or more, more preferably 1 mass% or more.

From the standpoint of preventing an increase in the viscosity of the ink, the concentration of the water insoluble polymer contained in the ink is preferably 8 mass% or less, more preferably 4 mass% or less.

The concentration of the water insoluble polymer contained in the ink is preferably 0.5 mass% or more and 8 mass% or less, more preferably 1 mass% or more and 4 mass% or less.

From the standpoint of having a sufficient concentration to achieve desired coloring, in other words, preventing the color from becoming lighter than the expected one, the concentration of the coloring material contained in the ink is preferably 2 mass% or more, more preferably 4 mass% or more.

From the standpoint of easily dispersing the pigment in the ink, the concentration of the coloring material contained in the ink is preferably 8 mass% or less, more preferably 7.5 mass% or less.

The concentration of the coloring material contained in the ink is preferably 2 mass% or more and 8 mass% or less, more preferably 4 mass% or more and 7.5 mass% or less.

The medium of the ink may be water or an organic solvent, or may be a mixture of them. The medium of the ink is preferably a substance in which a nanofiber has no solubility. From such a standpoint, for example, deionized water is a preferable medium. Using, as the medium of the ink, a substance in which a nanofiber has no solubility is advantageous because the nanofiber can maintain the shape of the fiber in the membrane-like region.

From the standpoint of preventing the clogging of a nozzle, the medium of the ink is preferably water or a mixture of water and a water soluble organic solvent, more preferably a combination of water and a polyol which is liquid at 20°C.

When the medium of the ink is a mixture of water and a water soluble organic solvent, the concentration of water in the ink is preferably 30 mass% or more, more preferably 50 mass% or more. When the nanofiber has the water insoluble polymer as the main component thereof, the concentration is even more preferably 70 mass% or more from the standpoint of the adhesion between the membrane-like region and the nanofiber.

When the medium of the ink is a mixture of water and a water soluble organic solvent, the concentration of water in the ink is preferably 90 mass% or less, more preferably 85 mass% or less, even more preferably 80 mass% or less, considering the balance with another component.

When a volatile organic solvent selected from ethanol and acetone is used as the water soluble organic solvent, the proportion of the volatile organic solvent relative to water is preferably 30 mass% or less, more preferably 10 mass% or less, even more preferably 1 mass% or less from the standpoint of preventing the clogging of a nozzle in inkjet printing.

Examples of the polyol contained in the aforesaid ink include glycerin, 1,3-butylene glycol, propylene glycol, and polyethylene glycol. One or two or more selected from them may be used.

When the nanofiber has the water insoluble polymer as a main component thereof, the concentration of the polyol contained in the ink is preferably less than 20 mass%, more preferably 18 mass% or less, from the standpoint of the fixing of a print in the nanofiber layer.

From the standpoint of preventing drying at the discharge pot of an ink, the ink preferably contains 5 mass% or more of the polyol.

The term "main component" as used herein means a component accounting for the maximum mass% among the accumulations in the nanofiber layer.

By printing on the nanofiber layer with the aforesaid ink by inkjet printing, a membrane-like region can be formed in the nanofiber layer. The nanofiber layer has a high capillary force because the fiber-to-fiber distance of the nanofibers constituting the nanofiber layer is short. Since the ink droplets sprayed to the nanofiber layer are reliably trapped between the nanofibers and at the same time, do not diffuse easily, strike-through of the ink is prevented and a membrane-like region is formed over the entire region in the thickness direction from one of the surfaces of the nanofiber layer to the other surface.

After printing is conducted on the nanofiber layer, the resulting nanofiber layer may be subjected to an additional step. For example, after printing on the nanofiber layer by the aforesaid various printing methods, a cover layer may be formed on the printed surface of the nanofiber layer.

The cover layer is formed to protect the printed nanofiber layer. Alternatively, the cover layer is formed to impart the nanofiber sheet of the present invention with an additional function.

To satisfy such a purpose, the cover layer may be a nanofiber layer. Since the cover layer is a nanofiber layer, the printed nanofiber layer is protected with the cover layer.

The cover layer may be an adhesive layer. The cover layer which is an adhesive layer can impart the nanofiber sheet of the present invention with adhesion properties.

When the cover layer is a nanofiber layer, this nanofiber layer may be formed, for example, by electrospinning or melt blowing. The nanofiber sheet manufactured using the aforesaid method has a structure in which a first nanofiber layer having a membrane-like region and a second nanofiber layer having no membrane-like region are stacked one after another. The second nanofiber layer is preferably formed by stacking nanofibers on the printed surface of the first nanofiber layer by electrospinning.

It is also preferred to form the nanofiber layer by forming a base layer and stacking nanofibers on one of the surfaces of the base layer by electrospinning. The base layer is a layer capable of retaining the shape of a nanofiber sheet and it may be either a single layer or a multilayer.

When a nanofiber layer is stacked on one of the surfaces of a base layer and then a second nanofiber layer is stacked thereon, the nanofiber layer stacked first, that is, the first nanofiber layer has, on a surface opposite to the surface where the base layer is present, the second nanofiber layer having no membrane-like region. A nanofiber sheet having such a multilayer structure may be attached to a surface of an object so that the first nanofiber layer is brought into contact with the surface or may be attached to a surface of an object so that the second nanofiber layer is brought into contact with the surface.

When the cover layer is an adhesive layer, the adhesive layer may be formed, for example, by electrospinning. A solvent used for a solution for forming the adhesive layer may be the same as the solvent for the nanofiber layer. Examples of the combination of the solvent used for a solution for forming the adhesive layer and the adhesive include a combination of ethyl acetate and an acrylic non-water soluble anionic resin and a combination of ethanol and an acrylic non-water soluble nonionic resin. In forming the adhesive layer by electrospinning, the spinning conditions similar to those for the formation of the nanofiber layer may be used.

The nanofiber layer whose membrane-like region is formed by inkjet printing can be formed by a nanofiber manufacturing technique known to date. A nanofiber layer made of the accumulation of nanofibers can be formed, for example, by electrospinning or melt blowing.

In electrospinning, a raw material liquid is discharged while applying a high voltage between a nozzle for discharging the raw material liquid and a collecting electrode placed opposite to the nozzle with a predetermined distance therebetween. Fibers having a smaller diameter are formed by cooling and solidifying the raw material liquid discharged from the nozzle while drawing by the Coulomb force.

In melt blowing, a molten resin discharged from a spinning nozzle is accompanied by a heated gas and thereby stretched in a fiber form and then, stacked on a collection net.

In the present invention, either electrospinning or melt blowing can be used, but electrospinning is preferred from the standpoint of obtaining fibers with a smaller diameter more easily.

When electrospinning is used for the formation of a nanofiber layer, nanofibers may be stacked on a collection net or nanofibers may be stacked on one of the surfaces of a base layer to form the nanofiber layer.

As the base layer, a synthetic resin film or a fiber layer other than a nanofiber layer may be used. As such a fiber layer, for example, a nonwoven fabric manufactured using various methods may be used. When a fiber layer is used as the base layer, the fiber diameter of fibers constituting the fiber layer is preferably larger than that in the region of nanofibers. For example, the average fiber diameter of the fiber layer is preferably larger than the average fiber diameter of the nanofiber layer.

The nanofiber sheet thus obtained has a base layer on one of the surfaces of the nanofiber layer. In the resulting nanofiber sheet, the nanofiber layer is preferably releasable from the base layer. This makes it easy to attach the nanofiber layer to the surface of an object.

A method of attaching the nanofiber layer includes a step of attaching a nanofiber sheet to an object and a step of applying a liquid to the nanofiber sheet or the object.

Described specifically, the nanofiber layer of the nanofiber sheet is brought into contact with the surface of an object to be attached to conform the nanofiber layer to the surface. In this case, a liquid is preferably applied to the surface of an object to be attached to wet the surface.

Next, the nanofiber layer is released from the base layer. While the nanofiber layer is left on the surface of the object to be attached, the base layer is removed. The nanofiber layer left on the surface of the object to be attached becomes transparent and a colored region formed in the nanofiber layer becomes visually clear.

By a series of these operations, the nanofiber layer can easily be attached to the surface of the object. The migration of the coloring material from the nanofiber layer thus attached hardly occurs. In addition, the nanofiber layer thus attached to the object does not peel off with a light force and at the same time, is released easily.

Another using method of the nanofiber sheet of the present invention is to apply a liquid to an object to be attached to wet it and attach the nanofiber sheet of the present invention to a site of the object to which the liquid is applied. In this case, the nanofiber layer of the nanofiber sheet is placed to face the object to be attached.

A further using method is to apply a liquid to the nanofiber sheet of the present invention to wet the sheet therewith and attach the nanofiber sheet to the object so that the surface of the nanofiber sheet to which the liquid is applied faces the object to be attached. Also in this case, the nanofiber layer of the nanofiber sheet is placed to face the object to be attached.

A still further using method is to attach the nanofiber sheet of the present invention to an object to be attached and then apply a liquid to the nanofiber sheet to wet the sheet therewith. Also in this case, the nanofiber layer of the nanofiber sheet is placed to face the object to be attached.

As the liquid, water or a mixture of water with a water soluble organic solvent may be used. As this mixture, for example, various cosmetic liquids are advantageously used.

The aforesaid various methods are advantageous because the nanofiber layer becomes more transparent and the colored region comprised of a water insoluble membrane-like region can be made clearer visually. When the membrane-like region contains a water insoluble polymer and at the same time, contains a pigment, the colored region can be effectively prevented from blurring even if it is brought into contact with a liquid, particularly, water.

Examples of the object to be attached include the human skin and nail and the rugged surface of an object such as walls, dishes, and plants such as branches and leaves.

In the nanofiber sheet having a multilayer structure including the aforesaid cover layer and nanofiber layer, the base layer may be placed on the side of the nanofiber layer having the membrane-like region formed therein. In this case, after the nanofiber layer is formed on one of the surfaces of the base layer and printing is given to the nanofiber layer to form a membrane-like region, a cover layer may be formed on the nanofiber layer.

In the nanofiber sheet of such a mode, after the cover layer is brought into contact with the surface of an object to be attached, the nanofiber layer is released from the base layer and thus the cover layer and the nanofiber layer may be attached to the surface of the object.

When the cover layer and the nanofiber layer are attached, the nanofiber layer having a membrane-like region formed therein is separated from the surface of the object by the cover layer, so that the nanofiber sheet has such an advantage that the coloring material contained in the membrane-like region does not easily mitigate to the surface of the object. When the object is the human skin, this advantage becomes higher from the standpoint of safety.

The base layer may be placed on the side of the cover layer. In this case, after the cover layer is formed on one of the surfaces of the base layer and then, the nanofiber layer is formed on the cover layer, printing is conducted on the nanofiber layer to form a membrane-like region in the nanofiber layer.

In the nanofiber sheet of the present invention, examples of the water insoluble polymer constituting the nanofiber include one or two or more selected from polyester-based resins, polyolefin-based resins, polyamide-based resins, fluorine-based resins, acrylic resins, styrene-based resins, urethane-based resins, vinyl-based resins, imide-based resins, silicone-based resins, and natural resins.

Examples of the polyester-based resins include one or two or more selected from PET, polytetramethylene terephthalate, PBT, PEN, polybutylene naphthalate, PHB, PHA, PBS, PLA, and PBN.

Examples of the polyolefin-based resins include one or two or more selected from polypropylene and polyethylene.

Examples of the polyamide-based resins include one or two or more selected from nylon and aramid.

Examples of the fluorine-based resins include one or two or more selected from PTFE, PCTFE, CTFE, PVDF, PVF, PFA, and FEP.

Examples of the vinyl-based resins include one or two or more selected from vinyl chloride resins, vinyl acetate resins, vinylidene chloride resins, completely saponified polyvinyl alcohol which can be insolubilized after fiber formation, partially saponified polyvinyl alcohol which can be subjected to a crosslinking treatment after fiber formation by using a crosslinking agent in combination, polyvinyl acetal, polyvinyl butyral, and polyvinyl acetal diethylamino acetate.

Examples of the imide-based resins include one or two or more selected from polyimide resins and polyamide-imide resins.

Examples of the silicone-based resins include one or two or more selected from silicone resins, and oxazoline-modified silicone resins such as poly(N-propanoylethyleneimine)-graft-dimethylsiloxane/$\gamma$-aminopropylmethylsiloxane copolymer.

Examples of the natural resins include one or two or more selected from Twain (main component of corn protein), rosin, shellac, collagen, gelatin, fibrin, and casein.

These water insoluble polymers may be used either singly or in combination of two or more. From the standpoint of stably and easily forming the nanofiber layer, a polyvinyl butyral resin is preferred.

On the other hand, examples of the water soluble polymer constituting the nanofiber include one or two or more selected from natural polymers and synthetic polymers.

Examples of the natural polymers include one or two or more selected from mucopolysaccharides such as pullulan, hyaluronic acid, chondroitin sulfate, poly-$\gamma$-glutamic acid, modified corn starch, $\beta$-glucan, glucooligosaccharide, heparin, and keratosulfate; cellulose, pectin, xylan, lignin, glucomannan, galacturon, psyllium seed gum, tamarind seed gum, gum arabic, tragacanth gum, soybean water-soluble polysaccharides, alginic acid, carrageenan, laminaran, agar (agarose), fucoidan, methyl cellulose, hydroxypropyl cellulose, and hydroxypropylmethyl cellulose.

Examples of the synthetic polymer include one or two or more selected from acrylic acid-based polymers, vinyl-based polymers, and ethylene glycol-based polymers.

Examples of the acrylic acid-based polymers include sodium polyacrylate.

Examples of the vinyl-based polymers include one or two or more selected from partially saponified polyvinyl alcohol (not used in combination with a crosslinking agent), low-saponified polyvinyl alcohol, and polyvinylpyrrolidone.

Examples of the ethylene glycol-based polymers include one or two or more selected from polyethylene glycol and polyethylene oxide.

Examples of the other water soluble polymer include one or two or more selected from water soluble nylons and water soluble polyesters.

These water soluble polymers may be used either singly or in combination of two or more.

The nanofiber sheet of the present invention is preferably attached to the human skin suitably for improving appearance, more preferably attached to a skin site having wrinkles or spots so that the sheet becomes wider than the skin site. The nanofiber sheet used in such a manner makes it possible to conceal wrinkles or spots present on the skin and improve the appearance of the skin. In short, the nanofiber sheet of the present invention is usable as a sheet for concealing wrinkles or spots.

In addition, the nanofiber sheet of the present invention is suited for use, for example, as a sheet for special makeup or sheet for body art.

Examples

The present invention will hereinafter be described more specifically by Examples, but the scope of the present invention is not limited by these Examples. Unless otherwise specified, "%" means "mass%".

[Example 1]

(1) Manufacture of nanofiber layer

As the base layer, "Bemliese (registered trademark) SE103", a nonwoven fabric of Asahi Kasei Corporation was used. A nanofiber layer was formed on one of the surfaces of the base layer by electrospinning. As a raw material liquid to be subjected to electrospinning, a mixture composed of 12% polyvinyl butyral, 6.8% 1,3-butanediol, and 81.2% ethanol was used. Electrospinning was conducted under the following conditions.

Applied voltage: 30 kV
Capillary-collector distance: 150 mm
Discharged amount of a solution: 3 mL/h
Atmosphere: 23°C, humidity: 40%RH

Under the aforesaid conditions, a nanofiber layer having an average fiber diameter of 700 nm and a basis weight of 3.2 g/m$^2$ was formed on the base layer. The ground color of the nanofiber layer thus obtained was white.

(2) Inkjet printing

Printing was conducted on the surface of the nanofiber layer with an inkjet printer. The inkjet printer used was "Handy Printer" (trade name), product of Ricoh Co., Ltd., and an alphabet letter in magenta color were printed on the nanofiber layer. An ink having a composition (mass%) of Ink 1 listed in the following Table 1 was used. In the table, the concentration of the pigment corresponds to the concentration of a coloring agent contained in the ink. In the table, a total concentration of all the polymers corresponds to the concentration of a water insoluble polymer contained in the ink.

A (coloring material)/polymer ratio in the nanofiber layer after printing was 2.3. The content of each component shown in Table 1 is an effective amount.

The nanofiber layer of the nanofiber sheet thus obtained had a thickness of 15 $\mu$m and a basis weight of 3.2 g/m$^2$.

[Example 2]

In the same manner as that used in Example 1 except that an alphabet letter in cyan color was printed on the nanofiber layer with an ink having a composition of Ink 2 listed in Table 1, (1) manufacture of a nanofiber layer and (2) inkjet printing were performed.

[Example 3]

In the same manner as that used in Example 1 except that an alphabet letter in yellow color was printed on the nanofiber layer with an ink having a composition of Ink 3 listed in Table 1, (1) manufacture of a nanofiber layer and (2) inkjet printing were performed.

[Comparative Example 1]

In a manner similar to that used in Example 1 except that an alphabet letter was printed on "Bemliese (registered trademark) SE103", a nonwoven fabric of Asahi Kasei Corporation which was made of 100% cuprammonium rayon and had an average fiber diameter was 15 $\mu$m, instead of on the nanofiber layer on which printing was performed in Example 1.

[Evaluation 1]

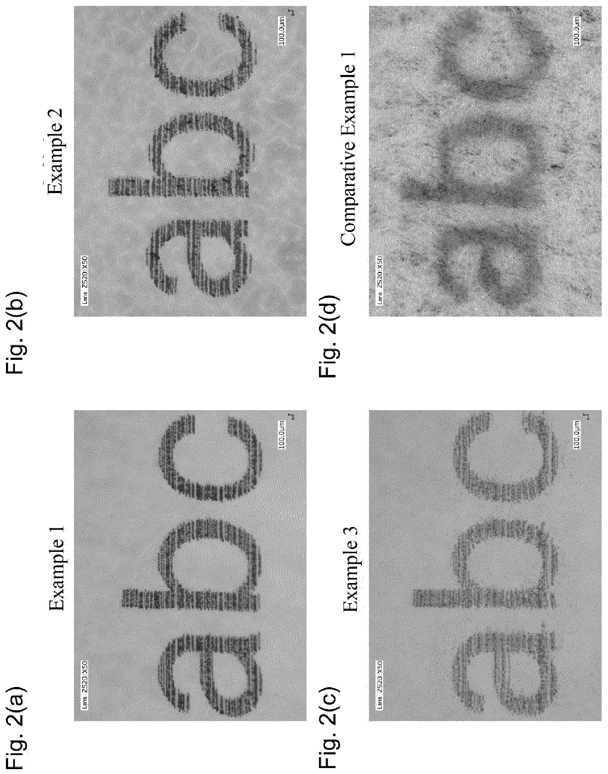

Printed states in Examples 1, 2, and 3 and Comparative Example 1 are photographed and shown in Figs. 2(a), (b), (c), and (d), respectively. Observation results of the printed states are shown in Table 2. In Table 2, the RGB value of the colored region and the RGB value of the ground color were measured under an illumination intensity of 200.

As is apparent from the comparison between Figs. 2(a) to (c) and Fig. 2(d), the alphabet letters are printed more clearly in Examples 1, 2, and 3 than in Comparative Example 1. This suggests that the colored site in the nanofiber sheet shows good color development.

Enlarged images of the printed alphabet letters are shown in Figs. 2(a), (b), (c), and (d). It has been found that in Examples 1, 2, and 3, each image has a colored region comprised of a plurality of membrane-like aggregate regions including a dot-like shape and the letters are comprised of a plurality of the colored regions. It has also been found that the membrane-like aggregate regions have a non-printed region therebetween. The average area of the membrane-like aggregate regions was calculated by the aforesaid arithmetic procedure of an average area and it was 1 332 $\mu$m$^2$ in Example 1, 1 232 $\mu$m$^2$ in Example 2, and 1 294 $\mu$m$^2$ in Example 3.

In contrast, the ink blurred and a dot-like printed portion was not observed in Comparative Example 1.

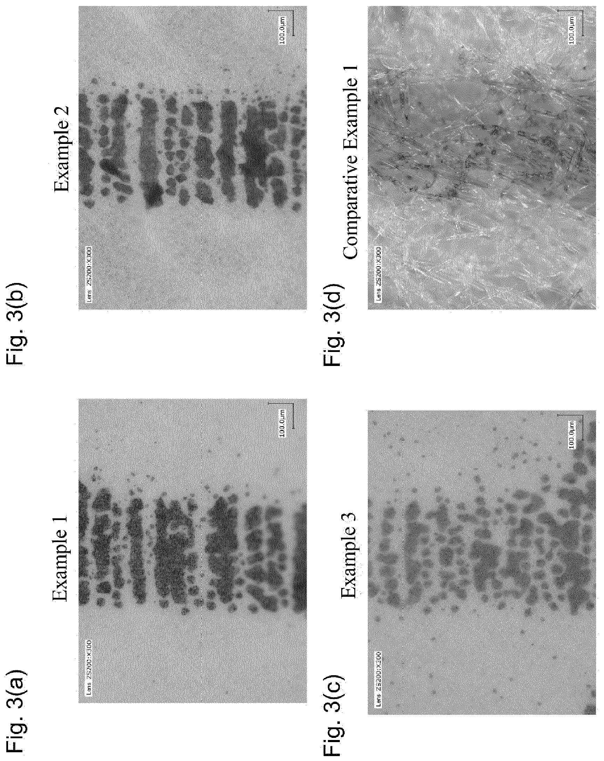

An enlarged image of Fig. 2(a) is shown in Fig. 3. As shown in this image, it has been found that each of the membrane-like aggregate regions is comprised of an aggregate of a plurality of membrane-like regions.

Further, the cross-section of the nanofiber layer in Example 1 was observed. Although not shown in the drawing, it has been found that the membrane-like region extended all over in the thickness direction from one of the surfaces to the other surface.

[Table 1]

| | | | Ink 1 | Ink 2 | Ink 3 |
|---|---|---|---|---|---|
| | | | Magenta | Cyan | Yellow |
| | | | 6111T (*1) | 6338JC (*2) | FY840T (*3) |
| Pigment dispersion in water | Pigment | | 7.00 | 4.00 | 5.00 |
| | Polymer | Benzyl acrylate (*6) | 1.20 | 0.69 | 0.86 |
| | Polymer | Styrene macromer (*7) | 0.31 | 0.17 | 0.22 |
| | Polymer | Methacrylic acid (*8) | 0.60 | 0.34 | 0.43 |
| | Polymer | NK ester EH-4E (*9) | 0.90 | 0.51 | 0.64 |
| | Sodium | | 0.16 | 0.09 | 0.11 |
| | Deionized water | | 41.20 | 23.60 | 29.50 |
| PEG400 | | Polyethylene glycol | 13.00 | 23.00 | 17.00 |
| OLFINE E1010 (*4) | Surfactant | Polyoxyethylene acetyl glycol ether | 0.50 | 0.50 | 0.50 |
| Proxel LVS (*5) (20%) | Antiseptic | 1,2-Benzoisothiazol 3 (2H)-one | 0.10 | 0.10 | 0.10 |
| Ion exchange water | | | Balance | Balance | Balance |

(continued)

|  |  | Ink 1 | Ink 2 | Ink 3 |
|---|---|---|---|---|
|  |  | Magenta | Cyan | Yellow |
|  |  | 6111T (*1) | 6338JC (*2) | FY840T (*3) |
|  | (Coloring material)/polymer ratio | 70/30 | 70/30 | 70/30 |

(*1) 6111T: "CFR6111T", magenta pigment, product of Dainichiseika Color & Chemicals
(*2) 6338JC: "CFB6338JC", cyan pigment, product of Dainichiseika Color & Chemicals
(*3) FY840T: "FY840T", yellow pigment, product of Dainichiseika Color & Chemicals
(*4) Polyoxyethylene acetyl glycol ether (number of moles of ethylene oxide added: 10), product of Nissin Chemical
(*5) 1,2-Benzoisothiazol 3(2H)-one (20%), product of Arch Chemicals Japan
(*6) "Viscoat#160", trade name; product of Osaka Organic Chemical Industry
(*7) "AS-6S", trade name; product of TOAGOSEI, number average molecular weight: 6000, segment: styrene-acrylonitrile, toluene solution, solid content: 51%
(*8) Methacrylic acid, trade name, product of Mitsubishi Chemical
(*9) 2-Ethylhexyl polyethylene glycol monomethacrylate, "EH4E", trade name, product of Shin-Nakamura Chemical (average number of moles of ethylene oxide added: 4, terminal: 2-ethylhexyl group)

[Table 2]

|  |  | Example 1 | Example 2 | Example 3 | Comp. Ex. 1 |
|---|---|---|---|---|---|
| State of membrane-like region |  | A plurality of membrane-like regions was found. | A plurality of membrane-like regions was found. | A plurality of membrane-like regions was found. | No membrane-like region was found. |
| Water insoluble polymer contained in membrane-like region |  | Benzyl acrylate | Benzyl acrylate | Benzyl acrylate | Benzyl acrylate |
|  |  | Styrene macromer | Styrene macromer | Styrene macromer | Styrene macromer |
|  |  | Methacrylic acid | Methacrylic acid | Methacrylic acid | Methacrylic acid |
|  |  | NK Ester EH-4E | NK Ester EH-4E | NK Ester EH-4E | NK Ester EH-4E |
| Pigment contained in membrane-like region |  | 6111T | 6338JC | FY860T | 6111T |
| Constituent member (main component) of nanofiber |  | PVB | PVB | PVB | Cuprammonium rayon |
| Difference between water insoluble polymer and constituent member of nanofiber |  | Kind of monomer unit | Kind of monomer unit | Kind of monomer unit | - |
| Main component of nanofiber |  | 99.9 | 99.9 | 99.9 | 100 |
| Peripheral region |  | Present | Present | Present | Absent |
| ∆RGB |  | 218 | 162 | 194 | - |
| RGB value (R1,G1,B1) of colored region |  | (117,84,122) | (102,150,110) | (69,119,171) | - |
| RGB value (R2,G2,B2) of ground color |  | (230,229,239) | (209,209,216) | (226,221,222) | - |

[Example 4]

The nanofiber sheet obtained in Example 1 was attached to the human skin.

First, the skin was wetted with water. Then, the nanofiber layer of the nanofiber sheet was brought into contact with the wetted skin. When the nanofiber layer conformed to the skin, the nanofiber layer was released from the base layer and the nanofiber layer was left on the skin. The nanofiber layer became transparent and the alphabet letter printed on the nanofiber layer was visually recognized clearly. Then, a cosmetic liquid (Rise Lotion II (refreshing type), trade name; product of Kao Corporation) was added dropwise onto the nanofiber layer. By the dropwise addition, the nanofiber layer became more transparent and the alphabet letter was visually recognized more clearly.

[Example 5]

The nanofiber sheet obtained in Example 1 was attached to the human skin.

First, the nanofiber layer of the nanofiber sheet obtained in Example 1 was wetted with water. Then, the nanofiber layer of the nanofiber sheet was brought into contact with the wetted skin. When the nanofiber layer conformed to the skin, the nanofiber layer was released from the base layer and the nanofiber layer was left on the skin. The nanofiber layer became transparent and the alphabet letter printed on the nanofiber layer was visually recognized clearly. Then, the cosmetic liquid same as that used in Example 4 was added dropwise onto the nanofiber layer. By the dropwise addition, the nanofiber layer became more transparent and the alphabet letter was visually recognized more clearly.

[Example 6]

The nanofiber sheet obtained in Example 1 was attached to the human skin.

First, the nanofiber layer of the nanofiber sheet was brought into contact with the skin. The nanofiber layer was released from the base layer and the nanofiber layer was left on the skin. Then, the nanofiber layer was wetted with water. The nanofiber layer became transparent and the alphabet letter printed on the nanofiber layer was visually recognized clearly. Then, the cosmetic liquid same as that used in Example 4 was added dropwise onto the nanofiber layer. By the dropwise addition, the nanofiber layer became more transparent and the alphabet letter was visually recognized more clearly.

[Example 7]

In the same manner with that used in Example 1 except that a 3.8 mm × 3.2 mm graphic (■) was printed on the nanofiber layer, (1) manufacture of the nanofiber layer and (2) inkjet printing were performed. A photographed printed state of Example 7 is shown in Fig. 5.

The nanofiber sheet thus obtained was attached to the human skin.

First, the skin was wetted with water. Then, the nanofiber layer of the nanofiber sheet was brought into contact with the wetted skin. When the nanofiber layer conformed to the skin, the nanofiber layer was released from the base layer and the nanofiber layer was left on the skin. The nanofiber layer became transparent and the graphic printed on the nanofiber layer was visually recognized clearly. Then, a cosmetic liquid (Rise Lotion II (refreshing type), trade name; product of Kao Corporation) was added dropwise onto the nanofiber layer. By the dropwise addition, the nanofiber layer became more transparent and the graphic was visually recognized more clearly.

[Comparative Example 2]

The nonwoven fabric obtained in Comparative Example 1 was attached to the human skin.

First, the skin was wetted with water. Then, the nonwoven fabric was brought into contact with the skin. The nonwoven fabric did not become transparent and the alphabet letter printed on the nonwoven fabric remained unclear. Then, a cosmetic liquid (Rise Lotion II (refreshing type), trade name; product of Kao Corporation) was added dropwise onto the nonwoven fabric, but the nonwoven fabric did not become transparent and the alphabet letter remained unclear.

In the aforesaid Examples and Comparative Examples, the ratio of the area change of the membrane-like region before and after immersion in deionized water was measured. In all the Examples and Comparative Examples, it was 90% or more. Printing was performed using a water soluble ink instead of Inks 1 to 3 listed in Table 1. In all the Examples and Comparative Examples, the ratio of the area change of the membrane-like region before and after immersion in deionized water was 0%.

Industrial Applicability

The present invention provides a nanofiber showing good color development at a colored site in a nanofiber sheet. The present invention also provides a method of manufacturing such a nanofiber sheet easily.

**Claims**

1. A nanofiber sheet having a nanofiber layer having an accumulation of nanofibers, and comprising a plurality of membrane-like regions extending between the nanofibers, wherein

   the membrane-like region contains a water insoluble polymer and a pigment, and
   the water insoluble polymer is different from a constituent member of the nanofibers.

2. The nanofiber sheet according to claim 1, wherein the nanofiber has a water soluble polymer or a water insoluble polymer.

3. The nanofiber sheet according to claim 1 or 2, wherein

   the nanofiber sheet has, in a planar view, a plurality of membrane-like aggregate regions,
   the membrane-like aggregate region is an aggregate of a plurality of the membrane-like regions which is adjacent to each other via a fiber, and
   the nanofiber sheet has a non-membrane-like region between the membrane-like aggregate regions adjacent to each other.

4. The nanofiber sheet according to claim 3, wherein
   in a planar view, the membrane-like aggregate regions have an average area, in a planar view, of 2 800 $\mu$m$^2$ or less.

5. The nanofiber sheet according to claim 3 or 4, wherein
   the membrane-like aggregate regions have an average area, in a planar view, of 300 $\mu$m$^2$ or more.

6. The nanofiber sheet according to any one of claims 1 to 5, wherein
   the membrane-like region extends all over a region in a thickness direction of the nanofiber layer.

7. The nanofiber sheet according to any one of claims 1 to 6, wherein
   the nanofiber has a size, in terms of a circle equivalent diameter, of 10 nm or more and 3000 nm or less.

8. The nanofiber sheet according to any one of claims 1 to 7, wherein
   the nanofiber layer is a porous member.

9. The nanofiber sheet according to any one of claims 1 to 8, wherein
   the nanofiber layer has a thickness of 50 $\mu$m or less.

10. The nanofiber sheet according to any one of claims 1 to 9, wherein
    the nanofiber layer has a thickness of 1 $\mu$m or more.

11. The nanofiber sheet according to any one of claims 1 to 10, wherein
    the nanofiber layer has a basis weight of 8 g/m$^2$ or less.

12. The nanofiber sheet according to any one of claims 1 to 11, wherein
    the nanofiber layer has a basis weight of 0.01 g/m$^2$ or more.

13. The nanofiber sheet according to any one of claims 1 to 12, wherein
    the sheet has a laminate of the nanofiber layer and a layer other than the nanofiber layer.

14. The nanofiber sheet according to any one of claims 1 to 13, wherein
    the sheet has a base layer on one of the surfaces of the nanofiber layer.

**15.** The nanofiber sheet according to claim 14, wherein
the nanofiber layer has a cover layer on a surface opposite to a surface where the base layer is present.

**16.** The nanofiber sheet according to any one of claims 1 to 15, which is a cosmetic sheet.

**17.** A method of using the nanofiber sheet according to any one of claims 1 to 16, comprising:

a step of attaching the nanofiber sheet to an object, and
a step of applying a liquid to the nanofiber sheet or the object.

**18.** The method of using the nanofiber sheet according to claim 17, wherein

the nanofiber layer has a base layer on one of surfaces thereof, and a cover layer on a surface opposite to the surface on which the base layer is present, and
the cover layer is attached to a surface of the object.

**19.** The method of using the nanofiber sheet according to claim 17 or 18, wherein

the liquid is applied to the object, and
the nanofiber sheet is attached to a site of the object to which the liquid is applied.

**20.** The method of using the nanofiber sheet according to claim 17, wherein

the liquid is applied to the nanofiber sheet, and
the nanofiber sheet is attached to the object so that the surface of the nanofiber sheet to which the liquid is applied faces the object.

**21.** The method of using the nanofiber sheet according to claim 17 or 18, wherein

the nanofiber sheet is attached to the object, and
the liquid is applied to the nanofiber sheet.

**22.** A method of manufacturing a nanofiber sheet, comprising:

forming a nanofiber layer having an accumulation of nanofibers by electrospinning, and
printing is performed on the nanofiber layer by inkjet printing with an ink containing a water insoluble polymer and a pigment to form a colored region
in the nanofiber layer, wherein

the water insoluble polymer is different from a constituent member of the nanofibers, and
a color of the colored region is different from a ground color of the accumulation of nanofibers.

**23.** The method of manufacturing a nanofiber sheet according to claim 22, wherein the ink used in inkjet printing contains a water insoluble polymer.

**24.** The method of manufacturing a nanofiber sheet according to claim 23, wherein
a concentration of the water insoluble polymer in the ink is 0.5 mass% or more.

**25.** The method of manufacturing a nanofiber sheet according to claim 23 or 24, wherein
a concentration of the water insoluble polymer in the ink is 8 mass% or less.

**26.** The method of manufacturing a nanofiber sheet according to any one of claims 22 to 25, wherein
a medium of the ink to be used in inkjet printing contains water.

**27.** The method of manufacturing a nanofiber sheet according to claim 26, wherein

the medium contains water and a polyol, and
the polyol is one or two or more selected from glycerin, 1,3-butyl alcohol, propylene glycol, or polyethylene glycol.

**28.** The method of manufacturing a nanofiber sheet according to claim 27, wherein
the nanofibers have a water insoluble polymer as a main component and the polyol contained in the ink has a concentration of less than 20 mass% in the ink.

**29.** The method of manufacturing a nanofiber sheet according to any one of claims 26 to 28, wherein

the medium of the ink to be used in inkjet printing is a mixture of water and a water soluble organic solvent, and the concentration of water in the ink is 30 mass% or more.

**30.** The method of manufacturing a nanofiber sheet according to any one of claims 26 to 29, wherein

a medium of the ink to be used in inkjet printing is a mixture of water and a water soluble organic solvent,
the nanofibers have a water insoluble polymer as a main component, and
the concentration of water in the ink is 70 mass% or more.

**31.** The method of manufacturing a nanofiber sheet according to any one of claims 26 to 30, wherein

a medium of the ink to be used in inkjet printing is a mixture of water and a water soluble organic solvent, and the concentration of water in the ink is 90 mass% or less.

**32.** The method of manufacturing a nanofiber sheet according to any one of claims 22 to 31, comprising:

forming a base layer, and
forming, on one of the surfaces of the base layer, the nanofiber layer having
the accumulation of nanofibers by electrospinning.

**33.** The method of manufacturing a nanofiber sheet according to any one of claims 22 to 32, wherein
after printing on the nanofiber layer, a cover layer is formed on a printed surface of the nanofiber layer.

**34.** The method of manufacturing a nanofiber sheet according to claim 33, wherein
a second nanofiber layer is formed as the cover layer by electrospinning.

Fig. 1(a)

F1

M1

F2

Fig. 1(b)

F3

F1

M2

F2

Fig. 1(c)

F1

M3  F2

Fig. 1(d)

M2  M1

M1

M1

M1

M3

Fig. 2(a)

Example 1

Fig. 2(b)

Example 2

Fig. 2(c)

Example 3

Fig. 2(d)

Comparative Example 1

Fig. 3(a)

Example 1

Fig. 3(b)

Example 2

Fig. 3(c)

Example 3

Fig. 3(d)

Comparative Example 1

Fig. 4

Example 1

Membrane-like aggregate region

Fig. 5

## INTERNATIONAL SEARCH REPORT

| International application No. |
|---|
| PCT/JP2020/047982 |

### A. CLASSIFICATION OF SUBJECT MATTER

D04H 1/728(2012.01)i; A61K 8/02(2006.01)i; A61K 8/72(2006.01)i; A61Q 1/00(2006.01)i

FI: D04H1/728; A61K8/02; A61K8/72; A61Q1/00

According to International Patent Classification (IPC) or to both national classification and IPC

### B. FIELDS SEARCHED

Minimum documentation searched (classification system followed by classification symbols)

D04H1/728; A61K8/02; A61K8/72; A61Q1/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2019/009002 A1 (PANASONIC IP MANAGEMENT CO., LTD.) 10 January 2019 (2019-01-10) claims, paragraphs [0001], [0033], [0035], [0046], [0083], [0084], [0087], examples | 1–21 |
| Y | claims, paragraphs [0033], [0083], [0084], examples | 22–34 |
| Y | JP 2019-167640 A (PANASONIC IP MANAGEMENT CO., LTD.) 03 October 2019 (2019-10-03) claims, paragraphs [0031], [0032], [0038], [0050] | 22–34 |
| Y | JP 2013-136550 A (KAO CORP.) 11 July 2013 (2013-07-11) claims, paragraphs [0001], [0048], examples | 22–34 |
| A | JP 2014-073984 A (KEYTRADING CO., LTD.) 24 April 2014 (2014-04-24) | 1–34 |

☐ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 March 2021 (02.03.2021) | 23 March 2021 (23.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

Information on patent family members

International application No.

PCT/JP2020/047982

| Patent Documents referred in the Report | Publication Date | Patent Family | Publication Date |
|---|---|---|---|
| WO 2019/009002 A1 | 10 Jan. 2019 | US 2020/0121573 A1 claims, paragraphs [0001], [0039], [0052], [0089], [0090], examples EP 3650006 A1 CN 110769807 A | |
| JP 2019-167640 A | 03 Oct. 2019 | (Family: none) | |
| JP 2013-136550 A | 11 Jul. 2013 | (Family: none) | |
| JP 2014-073984 A | 24 Apr. 2014 | (Family: none) | |

Form PCT/ISA/210 (patent family annex) (January 2015)

**EP 4 083 292 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013142852 A1
- JP 2019231573 A